# EUROPEAN PATENT APPLICATION

(11) **EP 4 413 948 A1**
(43) Date of publication of application: **14.08.2024**
(21) Application number: 22879004.4
(22) Date of filing: 20.10.2022
(51) Int. Cl.: A61C 5/64, B01F 101/19, A61M 5/178

(54) **SINGLE-USE SYRINGE FOR A DUAL-COMPONENT DENTAL MATERIAL**

(30) Priority: 06.10.2021 RU 2021129146
(71) Applicant: Sharafutdinova, El'vira Nurfikatovna, Cherdakly, 433400 (RU)
(72) Inventor: Sharafutdinova, El'vira Nurfikatovna, Cherdakly, 433400 (RU)
(74) Representative: JWP Patent & Trademark Attorneys
(86) International application number: PCT/RU2022/000316
(87) International publication number: WO 2023/059220

(57) **Abstract**

A single-use syringe intended for mixing and dispensing dual-component dental materials. The syringe comprises two cylindrical housings and a tubular cannula connected to said housings via a mixing chamber. Disposed in the cavities of said housings are pistons with rods. The ends of the rods are joined by an end connector for finger support. The pistons are provided with O-rings made of an elastic material. Said rings are disposed at the ends of the pistons facing the mixing chamber. Pointed tubular elements arranged in the mixing chamber are designed to pierce the ends of outlet channels of the cavities of the cylindrical housings. The other ends of the pistons are provided with conical guide recesses for orienting tapered ends of the rods, said rods also being provided with annular shoulders for interacting with the end walls of the cylindrical housings. Each cylindrical housing has, at the end facing the mixing chamber, an inner annular retaining groove for an O-ring, the diameter of which is greater than half the depth of the groove.

## Description

The present group of inventions pertains to the domain of medicine and medical technology, specifically addressing the mixing and distribution of bigradient dental materials.

Conventionally, a syringe designed for dispensing bigradient dental material consists of a tubular cannula connected to a mixing chamber, which in turn connects to a container housing two cylindrical bodies. These bodies encase pistons and rods, all interconnected by a finger-operated thrust end bridge (US 2003,0187387 A1, October 2, 2003). However, a notable drawback of this conventional syringe lies in the intricacy of its design, particularly the container's configuration, which serves as a common body for the cylindrical cases and necessitates a retainer for securing its connection to said cylindrical bodies.

A refinement of this initial concept emerges in the form of a disposable syringe for bigradient dental material, as detailed in US patent US 6936033 B2, dated August 30, 2005. This prototype retains the fundamental structure of its predecessor, featuring a tubular cannula linked to a mixing chamber, further connecting to two cylindrical bodies housing cavities accommodating pistons and rods, all linked via a finger-operated thrust end bridge.

Yet, the disadvantage of this prototype lies not in the mechanism itself, but rather in the way it is engineered to hinder reuse by complicating the process of disassembling it into individual components. However, this mechanism fails to fully deter reuse, as certain elements remain susceptible to repurposing within the confines of the established design.

The overarching objective of this group of inventions is to tackle the technical quandary posed by the limitations of existing devices.

The technical advancement achieved by this group of inventions manifests in the enhanced ease of manual dispensation inherent in a disposable syringe, facilitating the simultaneous mixing of two dental material components immediately prior to application in the oral cavity.

At its core, this group of inventions embodies a distinct configuration, comprising the disposable syringe for bigradient dental material. This syringe features the tubular cannula connected by the mixing chamber to two cylindrical bodies housing pistons and rods placed within their respective cavities connected by the thrust end bridge for fingers. The difference from the closest analogue lies in the fact that the pistons are equipped with O-rings made of elastic material located at the ends of the pistons and oriented towards the mixing chamber. Additionally, pointed tubular elements are integrated into the mixing chamber, designed to puncture the ends of the exit channels within the cylindrical bodies' cavities. The opposite ends of the pistons feature cone-shaped guide cavities, serving to orientate the tapering ends of the rods. These rods, in turn, are equipped with annular stops intended for interaction with the end walls of the cylindrical bodies, situated at holes reserved for the rods. Notably, the diameters of these holes are smaller than those of the ring stops on at least one end wall, thus ensuring a secure fit. Each cylindrical body, oriented towards the mixing chamber, is fashioned with an annular internal locking recess designed to accommodate an O-ring, with a diameter exceeding half the depth of the recess. Moreover, the cavities of each body are filled with a gradient of dental material, sufficient for at least one dose of the desired bigradient dental material.

The tubular cannula of the disposable syringe contains the mixing chamber equipped with pointed tubular elements, engineered to puncture the ends of the exit channels within the cylindrical bodies' cavities. This mixing chamber features threading for seamless connection to the two cylindrical syringe bodies, with the pointed tubular elements securely affixed to a liner within the mixing chamber, enabling rotational movement relative to its inner surface.

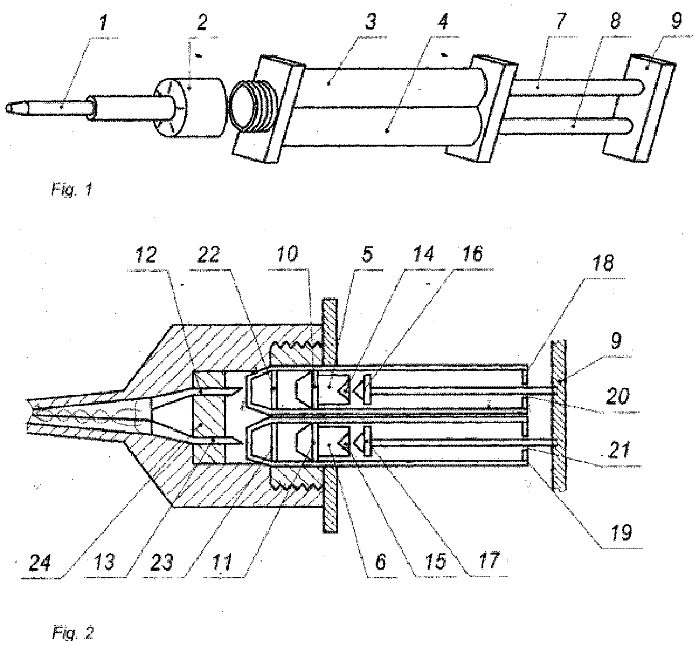

The essence of the group of inventions is provided in the accompanying drawing. FIG. 1 illustrates a general overview of the disposable syringe for bigradient dental material, while FIG. 2 presents a longitudinal incision, further detailing its internal structure.

The disposable syringe for administering bigradient dental material comprises the tubular cannula (1) connected to the mixing chamber (2), which, in turn, interfaces with two cylindrical bodies (3 and 4). Within the cavities of these bodies reside pistons (5 and 6), tethered to rods (7 and 8) and linked by the finger stop bridge (9). Notably, the pistons (5 and 6) are outfitted with O-rings (10 and 11) made from elastic material, situated at their ends oriented towards the mixing chamber (2).

Integrated into the mixing chamber (2) are pointed tubular elements (12 and 13), strategically positioned to puncture the ends of the outlet channels within the cavities of the cylindrical bodies (3 and 4). At the opposite ends, the pistons (5 and 6) feature cone-shaped guide cavities (14 and 15) designed to align with the tapering ends of the rods (7 and 8). These rods are equipped with ring stops (16 and 17) intended for interaction with the end walls (18 and 19) of the cylindrical bodies (3 and 4) at the rod holes (20 and 21). Noteworthy is the configuration ensuring that the diameters of these rod holes (20 and 21) are smaller than those of the annular stops (16 and 17) by a margin exceeding the thickness of one end wall (18 or 19).

Each cylindrical body (3 or 4), oriented towards the mixing chamber (2), features an annular inner locking notch (22 or 23) designed to accommodate the O-ring (10 or 11) with a diameter exceeding half the depth of the respective notch (22 or 23). Furthermore, the cavities within the cylindrical bodies (3 and 4) are partially filled with gradients of dental material, intended for a single dose or the required number of doses.

The tubular cannula (1) of the disposable syringe comprises the mixing chamber (2) with pointed tubular elements (12 and 13), which are designed to puncture the ends of the outlet channels within the cavities of the cylindrical bodies (3 and 4). The mixing chamber (2) features threading to facilitate seamless connection to the two cylindrical bodies (3 and 4). Notably, the pointed tubular elements (12 and 13) are affixed to the liner (24) securely mounted within the mixing chamber (2), allowing for rotational movement relative to its inner surface.

The disposable syringe intended for dispensing bigradient dental material, once prepared and enclosed within a sterile package, must contain the requisite volumes of gradients. These gradients, partially housed within bodies (3 and 4), incorporate pistons (5 and 6) and rods (7 and 8) installed during the syringe's manufacturing process. The tapered ends of the rods (7 and 8) rest within cone-shaped guide cavities (14 and 15), essential for orienting them, as they lack a permanent connection to the pistons (5 and 6). Upon preparation, the tubular cannula (1) is connected to the cylindrical bodies (3 and 4) in a predetermined position, with the pointed tubular elements (12 and 13) within the mixing chamber (2) delicately positioned, ensuring no contact with the outlet channels of the cylindrical bodies (3 and 4). To administer the bigradient dental material, the dentist retrieves the syringe from its packaging and proceeds to rotate the tubular cannula (1) several turns, securely fastening it onto the bodies (3 and 4). As the tubular cannula and the mixing chamber (2) rotate, the liner (24) within the mixing chamber (2), capable of rotational movement relative to its inner surface, maintains its position relative to the bodies (3 and 4). Consequently, upon convergence of the mixing chamber (2) and the bodies (3 and 4), the liner (24) pierces the ends of the outlet channels within the cavities of the cylindrical bodies (3 and 4).

By exerting pressure on the finger stop bridge (9), the dentist propels the rods (7 and 8), thereby advancing the pistons (5 and 6) within the cylindrical housings (3 and 4), extruding the gradients of dental material from each housing. This movement continues until the end surfaces of the pistons (5 and 6) come to rest against the ends of the cylindrical bodies (3 and 4). During this final stroke, the elastic O-rings (10 and 11) on the pistons (5 and 6) descend into the annular internal locking recesses (22 or 23), their diameters exceeding half the depth of the respective recesses (22 or 23), even before their end surfaces fully engage the ends of the cylindrical bodies (3 and 4).

The proportionate sizing of the O-rings (10 and 11) relative to the depths of the recesses (22 or 23), combined with the absence of a fixed connection between the tapered guide cavities (14 and 15) and the tapering ends of the rods (7 and 8), effectively precludes the retraction of the pistons (5 and 6) and thereby prevents syringe reuse. Additionally, the annular stops (16 and 17) prevent the removal of the rods (7 and 8) from the cylindrical bodies (3 and 4), interacting with the end walls (18 and 19) at the rod holes (20 and 21). Given that the diameters of these holes (20 and 21) are smaller than those of the annular stops (16 and 17) by a margin exceeding the thickness of one end wall (18 or 19), attempting to extract the rods (7 and 8) would inevitably result in the destruction of the end walls (18 and 19) of the cylindrical bodies (3 and 4).

### Example.

Patient A., 37 years old, presents with complaints of a cavity in the area of the 46th tooth, accompanied by short-term pain triggered by exposure to cold and sweet stimuli.

Upon examination, a carious lesion of moderate depth is noted on the masticatory surface of the 46th tooth. Sensitivity is elicited upon probing along the enamel-dentine border, with cold thermometry causing transient, painful sensations. However, percussion elicits no pain, and the gingival mucosa appears hyperemic.
Diagnosis: Dentin caries of the 46 tooth

### Treatment:

Anesthesia 1.7 ml of Ultracaini solution. To ensure optimal treatment outcomes, it is imperative to maintain a dry operating field devoid of moisture, foreign fluids, and saliva, facilitating the adhesion of composite and photopolymer materials to the tooth walls. Following anesthesia administration, the clinician proceeds to establish a protective barrier. This entails retrieving a disposable syringe from its sterile packaging and applying bigradient dental material, comprising a liquid silicone corrective impression compound and an activator. Subsequently, a fabric cofferdam and clamp are meticulously placed. Utilizing the disposable syringe, the remaining mixture is then injected directly into the interdental spaces, cervical region, and onto the fabric cofferdam adjacent to the tooth neck. Thanks to its flexible consistency, the silicone compound effortlessly conforms to and fills the interdental gaps and spaces between the tooth neck and the fabric cofferdam, ensuring a secure and dry working environment. Upon completion of treatment procedures, the hardened silicone mixture exhibits non-adhesive properties to the gum and tooth surfaces, facilitating easy removal without causing harm to the gingiva, tooth cervical zone, or dental papilla.

## Claims

1. The disposable syringe for dispensing bigradient dental material containing the tubular cannula connected to the mixing chamber, which in turn interfaces with two cylindrical bodies housing pistons and rods within cavities. These components are linked by the finger-operated thrust end bridge. The distinguishing feature of this syringe lies in the incorporation of O-rings made of elastic material situated at the ends of the pistons, oriented towards the mixing chamber. Additionally, pointed tubular elements within the mixing chamber are designed to puncture the ends of the outlet channels within the cavities of the cylindrical bodies. The opposite ends of the pistons are equipped with cone-shaped guide cavities to facilitate the orientation of the tapering ends of the rods. These rods are fitted with annular stops, intended for interaction with the end walls of the cylindrical bodies at rod holes, with diameters smaller than those of the annular stops by a margin exceeding the thickness of one end wall. Each cylindrical body, oriented towards the mixing chamber, features the annular internal locking recess designed to accommodate the O-ring, the diameter of which surpasses half the depth of the respective recess. Furthermore, the cavities of each body are filled with a gradient of dental material sufficient for at least one dose of bigradient dental material.

2. The tubular cannula designed for a single-use syringe according to Claim 1, comprising the mixing chamber equipped with pointed tubular elements intended to pierce the ends of the outlet channels within the cavities of the cylindrical bodies. This mixing chamber is threaded to facilitate connection to the two cylindrical syringe bodies. Notably, the pointed tubular elements are affixed to the liner mounted within the mixing chamber, capable of rotational movement relative to its inner surface.
